(19) **Europäisches Patentamt**

**European Patent Office**

**Office européen des brevets**

(11) **EP 1 134 289 A1**

(12) **EUROPEAN PATENT APPLICATION**
published in accordance with Art. 158(3) EPC

(43) Date of publication:
**19.09.2001 Bulletin 2001/38**

(51) Int Cl.⁷: **C12Q 1/02**, A61K 45/00,
A61P 31/00, A61P 37/04

(21) Application number: **99972691.2**

(22) Date of filing: **24.11.1999**

(86) International application number:
**PCT/JP99/06537**

(87) International publication number:
**WO 00/31288 (02.06.2000 Gazette 2000/22)**

(84) Designated Contracting States:
**AT BE CH CY DE DK ES FI FR GB GR IE IT LI LU MC NL PT SE**
Designated Extension States:
**AL LT LV MK RO SI**

(30) Priority: **25.11.1998 JP 33427098**

(71) Applicant: **Takeda Chemical Industries, Ltd.**
**Osaka-shi, Osaka 541-0045 (JP)**

(72) Inventors:
• **TAKASHIMA, Katsunori**
**Yao-shi, Osaka 581-0051 (JP)**
• **IIZAWA, Yuji**
**Muko-shi, Kyoto 617-0003 (JP)**

(74) Representative:
**Helbing, Jörg, Dr. Dipl.-Chem. et al**
**Patentanwälte**
**von Kreisler-Selting-Werner,**
**Postfach 10 22 41**
**50462 Köln (DE)**

(54) **METHOD FOR SCREENING CD14 AGONIST OR ANTAGONIST**

(57) Compounds which change the binding property of lipopolysaccharides to CD14 can be screened efficiently by using labeled lipopolysaccharides and cells in which a membrane-bound CD14 is expressed.

**Description**

Technical Field

The present invention provides a highly sensitive screening method for efficiently finding compounds that change the binding properties of lipopolysaccharides to CD14.

That is, the invention provides (1) a method' for screening for compounds which change the binding property of lipopolysaccharides to CD14, (2) a kit for the screening, (3) compounds which are obtained using said screening method or screening kit and change the binding property of lipopolysaccharides to CD14 (for example, compounds which interfere with the binding of microbial components released by bacterial infection to a membrane-binding CD14 to inhibit activation of cells, i.e., CD14 antagonists, etc.), and (4) pharmaceut-ical preparations which comprise drugs comprising a compound or compounds which change the binding property of lipopolysaccharides to CD14 (for example, drugs for treating septic shock containing the above antagonists), etc.

Background Art

Among syndromes accompanied by systemic inflammatory reactions (systemic inflammatory response syndrome (SIRS)) which are caused by an improper or excessive reaction in the living body against severe invasion, the most prominent pathologic expression is septic shock, particularly shock in serious infectious diseases caused by gram-negative bacteria, which shock is caused by lipopolysaccharides (LPS) which is endotoxin released from the lysate during administration of antimicrobial agents. This septic shock has become a very serious problem in therapy with antimicrobial agents.

Septic shock is caused particularly by activation of monocyte-type cells by microbial components released during bacterial infections and by excessive production of a variety of mediators from these cells. In order that the microbial components released during bacterial infections activate a target cell such as macrophage, it is necessary that the microbial components are recognized by the target cell receptor. In a recent study, it was found that a differentiation antigen of macrophage/monocyte, i.e., CD14, plays an important role in recognizing the microbial components by the target cell (Science, 1990, 249, 1431). CD14 is present in two configurations, i.e., membrane-bound type (mCD14) and soluble type (sCD14). mCD14, which has no membrane-penetrable domain, is an anchor protein attached on the cell surface with glycosylphosphatidylinositol. On the other hand, sCD14, which is present in sera, is either synthesized in the liver or cleaved from the surface of macrophage, monocyte, etc., by action of phosphatidylinositol-specific phospholipase (Mol. Immunol., 1989, 26, 657).

In binding of the microbial components to mCD14 in the case of gram-negative bacteria, LPS, one of the microbial components, forms a complex with an LPS-binding protein (LBP) which is an acute stage protein in sera. LBP works catalytically to promote the binding of LPS to mCD14 (Annu. Rev. Immunol., 1995, 13, 437).

A variety of mediators such as cytokines, e.g., TNF-$\alpha$, IL-1, IL-6, IL-8, etc., nitrogen monoxide (NO), superoxide, platelet activating factor (PAF), arachidonate cascade product, etc. are produced from the cells activated by the microbial components (Clin. Infect. Dis., 1994, 18 (Suppl. 2), S205). These mediators, respectively, express a plurality of activities, which are associated with each other to make the expression more complicated, and give damaging effect to the living body when the activity is expressed excessively. As a result, serious and multifarious clinical conditions such as hypotension, disseminated intravascular coagulation (DIC), adult respiratory distress syndrome (ARDS), respiratory insufficiency, multiple organ dysfunction (MODS), etc., are generated (Clin. Infect. Dis., 1994, 18 (Suppl. 2), S205).

As described above, the septic shock is caused by activation of monocyte-type cells with microbial components released from bacteria during the infection and by excessive production of a variety of mediators from the cells (Clin. Infect. Dis., 1994, 18 (Suppl. 2), S205); as for remedies, a method for blocking the factors of bacteria and that for blocking those of host cells have been investigated.

As therapies based on blocking the bacterial factors derived from gram-negative bacteria, the following have been investigated: (1) a method of using anti-endotoxin monoclonal antibodies (Infect. Immun., 1993, 61, 3863; JAMA, 1991, 266, 1097); (2) a method of using BPI (Bactericidal Permeability-Increasing Protein) (Antimicrob. Agents Chemother., 1996, 40, 65); (3) a method of using endotoxin antagonists (Science, 1995, 268, 80, WO9639411); (4) a method of using polymyxin B (the 36th ICAAC) ; (5) a method of using HDL (High-density Lipoprotein) (J. Exp. Med., 1996, 184, 1601); (6) a method of inhibiting LPS biosynthesis (Science, 1996, 274, 980).

As therapies based on blocking the host-cell factors (a variety of mediators such as TNF-$\alpha$, IL-1, NO, PAF, etc. overproduced by activation of macrophage), the following have been investigated: (1a) a method of using anti-TNF-$\alpha$ antibodies (Nature, 1987, 330, 662; Lancet, 1998, 351, 929); (1b) a method of using soluble TNF-$\alpha$ receptors (the 36th ICAAC); (1c) a method of inhibiting production of TNF-$\alpha$ (Nature, 1994, 370, 558; Nature, 1994, 370, 218); (2) a method of using IL-1 receptor antagonists (J. Exp. Med., 1991, 173, 1029; JAMA, 1994, 271, 1836) or a method of inhibiting an IL-1β converting enzyme (J. Antibiot., 1996, 49, 333); (3) a method of using anti-E-selectin antibodies (Inflamm. Res., 1996, 45, 448; Crit. Care. Med., 1996, 24, 229), etc.

Septic shock remedies by controlling the host-cell factors can be used in treatment of the shock caused by both gram-positive and gram-negative bacteria based on their action mechanism. Since a plurality of mediators are associated with each other in the septic shock to have various complicated activities, however, therapies by inhibiting a single mediator as described above were not effective in clinical trials. In addition, most septic shock remedies by controlling the bacterial factors target LPS of gram-negative bacteria, and the cell-activating factors of host are different between gram-positive and gram-negative bacteria. Accordingly, it is not possible to cure the septic shock caused by both gram-positive and gram-negative bacteria according to the method for controlling only the bacterial factors. It was found, however, that the bacterial components released from both infected gram-positive and gram-negative bacteria are bound to mCD14 present on the surface of target cells in the host and activate them (Immunity, 1994, 1, 509). Accordingly, the septic shock caused by both gram-positive and gram-negative bacteria may possibly be treated by drugs which interfere with the binding of the bacterial factors to mCD14 and inhibit activation of the cells.

It has been described in Infection and Immunity, June 1997, 2272-2277 that it was attempted to bind FITC (fluorescein isothiocyanate)-labeled LPS in which the ratio of FITC to LPS (FITC/LPS) is 1:0.9 to monocytes. It has also been described in Infection and Immunity, Feb. 1998, 486-491 that it was attempted to bind FITC-labeled LPS to THP-1 cells in which mCD14 was highly expressed by 1,25-dihydroxyvitamin $D_3$.

Disclosure of Invention

In view of the above, the present inventors worked assiduously to investigate these problems and found that compounds changing the binding properties of LPS to CD14, i.e., compounds changing fluorescence intensity of the FITC-labeled LPS as described below, can efficiently be screened according to a new screening method which comprises incubating human monocytes THP-1 in the presence of 1,25-dihydroxyvitamin $D_3$ to induce expression of mCD14, adding an FITC-labeled LPS together with a test compound to said monocytes in which expression of mCD14 has been induced, then after reaction completion, washing the cells, and detecting LPS that attached to the cells with measurement of fluorescence intensity. The invention was thus completed.

The invention relates to:

(1) a method for screening compounds changing the binding property of labeled lipopolysaccharide (hereinafter abbreviated to as LPS) to CD14 which comprises using a labeled LPS and cells in which a membrane-bound CD14 (hereinafter abbreviated to as mCD14) is expressed;

(2) a method for screening as described in the item (1), wherein LPS is one labeled with fluorescein isothiocyanate, biotin, enzyme or radioactive isotope;

(3) a method for screening as described in the item (1), wherein LPS is a rough form of LPS;

(4) a method for screening as described in the item (1), wherein LPS is one labeled with fluorescein isothiocyanate;

(5) a method for screening as described in the item (1), wherein an LPS labeled with fluorescein isothiocyanate in which the binding ratio (fluorescein isothiocyanate/LPS) is about 0.03 to about 1.0, and cells in which mCD14 is highly expressed are used;

(6) a method for screening as described in the item (1) or (5), wherein cells in which about 100,000 or more molecules of m CD14 per cell is expressed are used;

(7) a method for screening as described in the item (1), wherein the cells are those in which the expression of mCD14 is induced by 1,25-dihydroxyvitamin $D_3$;

(8) a method for screening as described in the item (1), wherein the cells are mononuclear leukocytes or polymorphonuclear leukocytes;

(9) a method for screening as described in the item (1), wherein the cells are monocytes, macrophages or neutrophils;

(10) a method for screening as described in the item (1), wherein the cells are human cells;

(11) a method for screening as described in the item (1), wherein the cells are THP-1;

(12) a method for screening as described in the item (1), wherein about 1 ng to about 30 ng of labeled LPS for $10^6$ cells are used;

(13) a method for screening as described in the item (1), which comprises measuring the binding property of the labeled LPS to mCD14, (i) when the labeled LPS is contacted with cells in which mCD14 is expressed, and (ii) when the labeled LPS and a test compound are contacted with cells in which mCD14 is expressed;

(14) a method for screening compounds changing the binding property of LPS to CD14 which comprises using flow cytometry, measuring (i) values of mean fluorescence intensity (MFI) when a labeled LPS is contacted with cells in which mCD14 is expressed; (ii) MFI value when the labeled LPS and a test compound are contacted with cells in which mCD14 is expressed; and (iii) MFI value when the labeled LPS and an anti-CD14 antibody are contacted with cells in which mCD14 is expressed, and calculating the inhibition rate from the following equation (I):

$$\text{Inhibition rate (\%)} = (1 - \text{MFI}^1/\text{MFI}^2) \times 100 \tag{I}$$

($\text{MFI}^1$ = MFI when a test compound is added - MFI when an anti-CD14 antibody is added; $\text{MFI}^2$ = MFI when a test compound is not added - MFI when an anti-CD14 antibody is added);

(15) a method for screening as described in the item (1), wherein the compound changing the binding property of LPS to CD14 is a CD14 antagonist;

(16) a method for screening as described in the item (1), wherein the compound changing the binding property of LPS to CD14 is a CD14 agonist;

(17) a method for screening (i) CD14 agonists, (ii) CD14 antagonists, (iii) compounds which are not bound to mCD14 but act on LPS, CD14 or LPS-binding protein (hereinafter abbreviated to as LBP) to inhibit the binding of LPS to CD14, or (iv) compounds which promote the binding of LPS to CD14, which comprises measuring the presence of the LPS-like action of compounds obtained by the method for screening as described in the item (13) or (14);

(18) a method for screening as described in the item (17), wherein the LPS-like action is production of TNF-α, IL-1, IL-6, IL-8, NO, superoxide, platelet activating factor, or arachidonate cascade product;

(19) a kit for screening compounds changing the binding property of LPS to CD14 which comprises a labeled LPS and cells in which mCD14 is expressed;

(20) a compound or derivative thereof changing the binding property of LPS to CD14, which is obtainable according to the method for screening as described in the item (1);

(21) (i) a CD14 agonist, (ii) a CD14 antagonist, (iii) a compound which is not bound to mCD14 but acts on LPS, CD14 or LBP to inhibit the binding of LPS to CD14, or (iv) a compound which promotes the binding of LPS to CD14, or a derivative thereof, which is obtainable according to the method for screening as described in the item (17);

(22) an agent for preventing or treating septic shock which comprises a compound or derivative thereof changing the binding property of LPS to CD14, which is obtainable according to the method for screening as described in the item (1);

(23) an agent for preventing or treating septic shock as described in the item (22), wherein the compound is a CD14 antagonist;

(24) an agent for preventing or treating septic shock as described in the item (22), wherein the compound is one which is not bound to mCD14 but acts on LPS, CD14 or LBP to inhibit the binding of LPS to CD14;

(25) an immunopotentiator which comprises a compound or derivative thereof changing the binding property of LPS to CD14, which is obtainable according to the method for screening as described in the item (1);

(26) an immunopotentiator as described in the item (25), wherein the compound is a CD14 agonist;

(27) an immunopotentiator as described in the item (25), wherein the compound is one which promotes the binding of LPS to CD14;

(28) a method for preventing or treating septic shock which comprises administering an effective amount of a compound or derivative thereof changing the binding property of LPS to CD14 to mammals, which is obtainable according to the method for screening as described in the item (1);

(29) a method for immunopotentiation which comprises administering an effective amount of a compound or derivative thereof changing the binding property of LPS to CD14 to mammals, which is obtainable according to the method for screening as described in the item (1);

(30) use of a compound or derivative thereof changing the binding property of LPS to CD14 for preparing an agent for preventing or treating septic shock, which is obtainable according to the method for screening as described in the item (1);

(31) use of a compound or derivative thereof changing the binding property of LPS to CD14 for preparing an agent for immunopotentiation, which compound can be obtained according to the method for screening as described in the item (1);

(32) A method for screening (i) CD14 agonist, (ii) a compound promoting the binding of LPS to CD14, (iii) CD14 antagonist, or (iv) a compound which is not bound to mCD14 but acts on LPS, CD14 or LBP to inhibit the binding of LPS to CD14, which comprises (A) measuring the binding property of labeled LPS to mCD14, (i) when the labeled LPS is contacted with cells in which mCD14 is expressed, and (ii) when the labeled LPS and a test compound are contacted with cells in which mCD14 is expressed; or

(B) using flow cytometry, measuring (i) values of mean fluorescence intensity (MFI) when a labeled LPS is contacted with cells in which mCD14 is expressed; (ii) MFI value when the labeled LPS and a test compound are contacted with cells in which mCD14 is expressed; and (iii) MFI value when the labeled LPS and an anti-CD14 antibody are contacted with cells in which mCD14 is expressed, and calculating the inhibition rate from the following equation (I):

$$\text{Inhibition rate (\%)} = (1 - MFI^1/MFI^2) \times 100 \tag{I}$$

($MFI^1$ = MFI when a test compound is added - MFI when an anti-CD14 antibody is added;
$MFI^2$ = MFI when a test compound is not added - MFI when an anti-CD14 antibody is added), thereby selecting a compound changing the binding property of LPS to CD14, and then measuring an activity inducing production of TNF-α, IL-1, IL-6, IL-8, NO, superoxide, platelet activating factor, or arachidonate cascade product for the selected compound;

> (a) selecting a compound having said induction activity for the production as (i) CD14 agonist or (ii) a compound promoting the binding of LPS to CD14; and
> (b) selecting a compound having no said induction activity for the production as (i) CD14 antagonist or (ii) a compound which is not bound to mCD14 but acts on LPS, CD14 or LBP to inhibit the binding of LPS to CD14;

(33) a compound or derivative thereof selected from a compound library prepared by means of a combinatorial chemistry technique according to the method for screening as described in the item (32), that is, (i) CD14 agonist, (ii) a compound promoting the binding of LPS to CD14, (iii) CD14 antagonist, or (iv) a compound which is not bound to mCD14 but acts on LPS, CD14 or LBP to inhibit the binding of LPS to CD14;
(34) an agent for immunopotentiation which comprises (i) CD14 agonist, (ii) a compound promoting the binding of LPS to CD14, or (iii) a derivative thereof, selected from a compound library prepared by means of a combinatorial chemistry technique according to the method for screening as described in the item (32);
(35) an agent for preventing or treating septic shock which comprises (i) CD14 antagonist, (ii) a compound which is not bound to mCD14 but acts on LPS, CD14 or LBP to inhibit the binding of LPS to CD14, or (iii) a derivative thereof, selected from a compound library prepared by means of a combinatorial chemistry technique according to the method for screening as described in the item (32).

Best Mode for Carrying Out the Invention

(1) Preparation of labeled LPS

In the present invention, any type of LPS derived from gram-negative bacteria such as Salmonella, Escherichia coli, etc., are used, with rough form ones being preferred. The rough form has no O-antigenic sugar chain and as its characteristics is lower in molecular weight than a smooth form. Practically, LPS derived from Salmonella, particularly those from Salmonella minnesota, especially Re595 (Sigma Chemical Co.) is preferably used. In addition, not only LPS themselves but also their various derivatives or isomers such as lipid A may also be used. These are hereinafter inclusively abbreviated as LPS.
In this invention, LPS needs to be labeled with a detectable labeling compound or radioactive isotope, etc., in a chemical or biochemical way. As the labeling compound, for example, FITC, biotin, enzyme, etc. are exemplified.
As FITC, for example a variety of derivatives (e.g., fluorescein-5-isothiocyanate (FITC isomer I), fluorescein-6-isothiocyanate (FITC isomer II), etc.) as well as FITC itself can be used. These are hereinafter merely abbreviated to as FITC. In this invention, FITC isomer I (Dojin Iyaku Co., Ltd.) is preferably used.
As biotin, for example, biotin-LC-hydrazide (biotinamido hexanoyl hydrazide), biotin-LC-ASA (1-(4-azidosalicylamide)-6-(biotinamido)-hexane), etc. are used.
As enzymes, for example, alkaline phosphatase, horseradish peroxidase, etc. are used.
As radioactive isotopes, for example, [$^3$H], [$^{51}$Cr], [$^{125}$I], etc. are used.
Labeling of LPS may be achieved in a per se known method. For example, when LPS is labeled with FITC, the labeling may be achieved in a method as described in Infection and Immunity, June 1997, 2272-2277, or its equivalent method. Practically, it may be performed in the following method or its equivalent method.
First, a base such as triethylamine, etc. is added to LPS in an amount of about 10 μl to about 2,000 μl, preferably about 100 μl to about 1,000 μl, more preferably about 500 μl, for 1 mg of LPS, and the mixture if required is applied to ultra-sonication. Then, a chelating agent such as ethylenediamine tetraacetate (EDTA) (preferably, 100mM EDTA) is added thereto in an amount of about 1 μl to about 200 μl, preferably about 10 μl to about 100 μl, more preferably about 50 μl. The mixture if required is applied to ultrasonication to disaggregate the aggregated LPS (preferably, converting into a monomer). Said ultrasonication is usually carried out at about 0°C to room temperature, preferably at about 4°C to about 20°C, particularly at about 4°C to about 15°C. As the time required for ultrasonication, preferably about 30 seconds to about 60 minutes, particularly about 1 minute to about 30 minutes, especially about 5 minutes to about 20 minutes, are preferred. The ultrasonication may be carried out only once either after addition of a base or after addition of a chelating agent, or respectively after addition of each reagent. Particularly, it is preferred to ultrasonicate for about 15 minutes after addition of the base and for about 5 minutes after addition of the chelating agent.

The mixture is then adjusted at pH about 3 to about 7, preferably about 4 to about 6, more preferably about 5, and then allowed to stand at a temperature of about 4°C to about 40°C, preferably about 4°C to about 30°C, more preferably at room temperature, for a period of about 30 seconds to about 15 minutes, preferably about 1 minute to about 10 minutes, more preferably about 1 minute to about 5 minutes, most preferably about 2 minutes. The reaction mixture is then neutralized with 1N sodium hydroxide, etc., to which FITC (the concentration of FITC being about 1 mg/ml to about 200 mg/ml, preferably about 5 mg/ml to about 100 mg/ml, more preferably about 25 mg/ml) dissolved in a proper buffer solution (e.g., borate buffer solution or carbonate/bicarbonate buffer solution, preferably carbonate/bicarbonate buffer solution, more preferably about 0.5M carbonate/bicarbonate buffer solution, etc.) at pH about 8 to about 11, preferably about 9 to about 10, more preferably about 9.5 is added. If required, the mixture is subjected to ultrasonication. The ultrasonication is carried out preferably at the same temperature as described in the above ultrasonication for a period of about 15 seconds to about 15 minutes, preferably about 30 seconds to about 5 minutes, more preferably about 1 minute to about 3 minutes, particularly about 1 minute. After ultrasonication, about 0.1% to about 10%, preferably about 1.6% (w/v) surface active agent (preferably deoxycholic acid salt (preferably sodium salt)) is added. The mixture is then incubated for a period of about 1 hour to about 48 hours, preferably about 6 hours to about 24 hours, more preferably about 18 hours, at a temperature of about 4°C to about 40°C, preferably room temperature to about 40°C, more preferably about 37°C, preferably under shading with stirring. After incubation, if required, in order to adjust the volume of the reaction mixture, distilled water or a proper buffer solution of pH about 3 to 11, preferably pH about 6 to about 10, which gives no effect on binding of LPS with FITC, is added. The mixture is then applied to dialysis with a permeable membrane, or to gel filtration using Sephadex G-25M (Pharmacia Biotech Co.) to remove the so-called free FITC which is not bound to LPS. For example, when a PD-10 column packed with Sephadex G-25M (Pharmacia Biotech Co.) is used, the volume of the reaction mixture is adjusted to 2.5 ml with distilled water and applied to the PD-10 column. Then, 3 ml of buffer solution for elution is applied, and the eluted fraction is collected as a fraction containing the FITC-labeled LPS. As the buffer solution used in equilibration of resin for gel filtration and elution, any of pH about 6 to about 8 may be used, and preferably PBS, more preferably Dulbecco's PBS (Dainippon Pharmaceutical Co., Ltd.) may be used. It is appropriate to add 0.01% to 1%, preferably 0.16% (each w/v) surface active agent (preferably deoxycholic acid salt (preferably sodium salt)) to said buffer solution.

The amount of FITC in the resulting FITC-labeled LPS can be determined by measuring at an excitation wavelength of 485 nm and a measuring wavelength of 538 nm using a fluorophotometer such as Fluoroskan II (Labsystems Co.).

In the above-mentioned FITC-labeled LPS, the ratio of binding of FITC to LPS (FITC/LPS) may preferably be established in the following (i) to (vi).

(i) About 0.03 - about 1.0
(ii) About 0.1 - about 1.0
(iii) About 0.3 - about 1.0
(iv) About 0.3 - about 0.8
(v) About 0.3 - about 0.5
(vi) About 0.3

Thus, establishment of the specific ratio of FITC to LPS increases sensitivity of measurement, which allows measurement at a lower concentration of LPS, that is, the experiment of binding can be achieved in a condition approximated to an LPS concentration in the living body. Accordingly, screening of the desired compounds can be made efficiently according to the screening method of the invention as described below, using such an FITC-labeled LPS.

(2) Preparation of cells in which mCD14 is expressed

As for the cells used in the present invention, any ones in which mCD14 is expressed on the cell surface may be employed, and those in which mCD14 is highly expressed are preferred. Practically, those in which mCD14 is expressed in an amount of about 100,000 molecules or more per cell may be used. Especially, those in which mCD14 of about 100,000 to about 2,000,000 molecules, preferably about 100, 000 to about 1, 500, 000 molecules, more preferably about 500,000 to about 1,500,000 molecules, particularly about 1,000,000 molecules per cell, is expressed are preferably used. In this invention, much more expressed cells may also be employed with no disturbance.

In addition to inherently highly expressive cells, cells in which the expression of mCD14 is induced can also be used preferably. Induction of the expression of mCD14 may be achieved with, for example, 1,25-dihydroxyvitamin $D_3$, etc. Said inducing agent may be used in an amount of about 1 ng to about 500 ng, preferably about 10 ng to about 100 ng, more preferably about 30 ng to about 40 ng for $3 \times 10^5$ cells.

As the species of the cells, for example, mononuclear leukocyte, polymorphonuclear leukocyte, etc. are used, with monocyte, macrophage, neutrophil, etc. being preferred. Particularly, monocyte is preferably used. As for said cells, any ones derived from mammals (e.g., guinea pig, murine, feline, canine, swine, sheep, bovine, monkey, human, etc.) may be used. Particularly, human cells such as THP-1, HL-60, U937, MonoMac-6, etc., are preferably used. Especially, THP-1 is preferably used.

The cell most preferably used in the invention is exemplified by THP-1 obtained in Reference Example 2 as described below, in which expression of mCD14 is induced by 1,25-dihydroxyvitamin D3. This cell expresses mCD14 at a rate of 1,000,000 molecules per cell (Journal of Leukocyte Biology, 61, June 1997, 721-728).

As described above, the cells in which the expression of CD14 is induced can be used in the invention, and the induction may be carried out in a method as described in Journal of Leukocyte Biology, 61, June 1997, 721-728 or its equivalent method.

For example, cells such as THP-1 (purchased from Dainippon Pharmaceutical Co., Ltd.) are dispersed into a proper culture medium (for example, RPMI medium 1640 with L-glutamine (GIBCO Co.) to which is inactivated fetal calf serum (GIBCO Co.) at a concentration of 10% (v/v) and penicillin-streptomycin at a concentration of 100 U/ml and 100 μg/ml, respectively as penicillin G sodium and streptomycin sulfate) at a content of about $3 \times 10^5$ cells/ml, and a proper amount of substance inducing expression of CD14 are added. Practically, for example, 1,25-dihydroxyvitamin $D_3$ (VD$_3$; Roussel-UCLAF S.A.) is added at a final concentration of about 10 nM to about 1,000 nM, preferably about 50 nM to about 500 nM, more preferably about 50 nM to about 200 nM, particularly about 100 nM. The mixture is then incubated, for example, in a $CO_2$ incubator in the presence of about 5% $CO_2$ at about 37°C, usually for a period of 1 to 7 days, preferably for 3 days to induce the expression of mCD14.

The amount of the expressed mCD14 can be determined using an antibody against CD14. As the antibody against CD14, commercially available one may be used, or it may be produced in a per se known method using CD14 as antigen.

Practically, the amount of the expressed mCD14 can be determined as follows. First, the cells in which the expression of CD14 are induced are suspended into PBS at about $10^6$ cells/ml, to which FITC-labeled anti-human CD14 monoclonal antibody clone UCHM1 (Ancell Co.) is added usually at a concentration of about 0.1 μg/ml to about 10 μg/ml, preferably about 1.6 μg/ml. The suspension is incubated usually at a temperature of about 4°C to about 25°C, preferably about 4°C to 15°C, more preferably about 4°C, usually for a period of about 15 minutes to about 24 hours, preferably about 15 minutes to about 12 hours, more preferably about 30 minutes to about 3 hours, particularly about 1 hour. The cells are usually washed 1 to 5 times, preferably 3 times with PBS by centrifugation (1,000 rpm, 3 minutes, 20°C), then suspended to a proper amount (for example, 1,000 μl) of PBS, and filtered through a tube with a filter (FALCON2235 (Becton Dickinson Co.)). For the cell suspension, the amount of the FITC-labeled anti-CD14 antibody usually bound to 10,000 cells is measured using a flow cytometer Cyto ACE-300 (Nippon Bunko Co.). As a buffer solution for elution, FACS Flow (Becton Dickinson Co.), etc. may be used.

(3) Screening method

The screening method of the invention is:

(Method A) A method for screening compounds changing the binding property of LPS to CD14 which comprises using a labeled LPS and cells in which mCD14 is expressed; preferably,
(Method B) a method for screening compounds changing the binding property of LPS to CD14 which comprises measuring the binding property of the labeled LPS to mCD14, (i) when the labeled LPS is contacted with cells in which mCD14 is expressed, and (ii) when the labeled LPS and a test compound are contacted with cells in which mCD14 is expressed; more preferably,
(Method C) a method for screening compounds changing the binding property of LPS to CD14 which comprises using flow cytometry, measuring (i) values of mean fluorescence intensity (MFI) when a labeled LPS is contacted with cells in which mCD14 is expressed; (ii) MFI value when the labeled LPS and a test compound are contacted with cells in which mCD14 is expressed; and (iii) MFI value when the labeled LPS and an anti-CD14 antibody are contacted with cells in which mCD14 is expressed, and calculating the inhibition rate from the following equation (I):

$$\text{Inhibition rate (\%)} = (1 - \text{MFI}^1/\text{MFI}^2) \times 100 \qquad \text{(I)}$$

(MFI[1] = MFI when a test compound is added - MFI when an anti-CD14 antibody is added;
MFI[2] = MFI when a test compound is not added - MFI when an anti-CD14 antibody is added).

In Method C, the resulting MFI value is regarded as the amount of the FITC-labeled LPS bound to the cells, and the activity of each test compound changing the specific binding property of the FITC-labeled LPS to mCD14 is calculated from the above equation (I).

In general, the screening of compounds changing the binding property of LPS to CD14 can be performed by examining the binding of labeled LPS to cells in which mCD14 is expressed, in the presence of a test compound. Practically, it may be achieved according to a method as described below or its equivalent method.

First, a test compound is added to cells in which mCD14 is expressed. The test compound is preferably dissolved in a proper buffer solution for screening. Such a buffer solution includes that which have no influence on the binding property of LPS to CD14, preferably those of pH about 6 to about 10, more preferably PBS, etc. (hereinafter referred to as a buffer solution such as PBS, etc.).

The solution for the test compound is added in an amount of about 1 $\mu$l to 1,000 $\mu$l, preferably about 5 $\mu$l to about 200 $\mu$l, particularly 5 $\mu$l to about 100 $\mu$l, especially about 20 $\mu$l, for about $10^6$ cells. The content of the test compound in the solution may be chosen properly.

Subsequently, if required, for example, the mixture is pre-incubated in the presence of about 5% $CO_2$ at about 37°C for about 1 minute to about 60 minutes, preferably about 5 minutes to about 30 minutes, more preferably about 15 minutes.

The FITC-labeled LPS is added in an amount of about 1 ng to about 1,000 ng, preferably about 1 ng to about 100 ng, more preferably about 1 ng to about 30 ng, particularly about 5 ng for about $10^6$ cells. Then, (i) human serum diluted about 1-fold to about 1, 000-fold, preferably about 2-fold to about 100-fold, more preferably about 5-fold to about 50-fold, particularly about 10-fold with a buffer solution such as PBS, etc., or (ii) about 1 ng to about 1,000 ng of LBP, preferably about 1 ng to about 100 ng, particularly about 20 ng is added. The mixture is incubated in the presence of about 5% $CO_2$ at about 37°C for about 15 minutes to about 12 hours, preferably about 15 minutes to about 2 hours, more preferably about 1 hour. The cells are washed several times, preferably 3 times with a buffer solution such as PBS, etc. using a cell washable equipment such as Multi-Screen Filtration System Vacuum Manifold (Millipore Co.), then suspended into 300 $\mu$l of PBS, and filtered. The resulting cell suspension is measured for the amount of FITC-labeled LPS bound to 1,000 cells using a flow cytometer such as CytoACE-300, etc. Usually, FACS Flow may be used as a buffer solution for elution.

When no test compound is added, that is, the determination of the maximum binding (negative control) may be achieved by adding a buffer solution such as PBS, etc., in place of the test compound, in an amount equal to that of the test compound.

Determination of the specific binding to mCD14 (positive control) may be achieved by adding an anti-CD14 antibody (for example, anti-human CD14 monoclonal antibody, practically clone MEM18 (SANBIO Co.), diluted with a buffer solution such as PBS, etc. about 1-fold to about 100-fold, preferably about 10-fold), in place of the test compound, in an amount equal to that of the test compound.

The resulting MFI value is regarded as the amount of the FITC-labeled LPS. bound to the cells, and the activity of each test compound changing the specific binding property of the FITC-labeled LPS to mCD14 can be calculated from the above equation (I) on the basis of the determined value.

The test compounds of which the inhibition rate is about 20% or higher, preferably about 50% or higher, may be chosen as compounds inhibiting the binding of LPS to CD14. On the other hand, the test compounds of which the inhibition rate is about -20% or less, preferably about -50% or less, may be chosen as compounds promoting the binding of LPS to CD14.

Thus, the screening method of the invention is useful in screening compounds which inhibit or promote the binding of LPS to CD14.

The compounds inhibiting the binding of LPS to CD14 include the following three classes of compounds.

(i) Compounds which bind to mCD14 to inhibit the binding of LPS to CD14, and have per se LPS-like effect (so-called CD14 agonist).
(ii) Compounds that bind to mCD14 to inhibit the binding of LPS to CD14, but themselves have no LPS-like effect (so-called CD14 antagonist).
(iii) Compounds which do not bind to mCD14, but act on CD14, LPS, LBP, etc., to inhibit the binding of LPS to CD14 (hereinafter abbreviated to as CD14 inhibitor).

The presence or absence of the binding of said compounds to mCD14 can be determined according to a conventional method for determining the binding activity of ligands to receptors, for example, a method as described in European Journal of Pharmacology, 1991, 208, 199-205 or its equivalent method.

The presence or absence of the LPS-like effect may be determined according to a per se known method, for example, a method as described in Antimicrob. Agents Chemother., 1998, 42, 1015-1021 or its equivalent method. As the LPS-like effect, for example, activities inducing production of cytokines, e.g., TNF-α, IL-1, IL-6, IL-8, etc., nitrogen monoxide (NO), superoxide, platelet activating factor, arachidonate cascade product, etc., are exemplified.

Accordingly, the screening method of the invention further provides a method for screening CD14 agonist, CD14 antagonist, CD14 inhibitor or a compound promoting the binding of LPS to CD14 (hereinafter, CD14 promoter) by determining (i) the presence of the binding of said compounds to mCD14 and (ii) the presence of the LPS-like effect for the compounds screened by the above Method A to Method C.

More particularly, the invention provides: a method for screening (i) CD14 agonist, (ii) CD14 promoter, (iii) CD14 antagonist, or (iv) CD14 inhibitor, which comprises (A) measuring binding property of the labeled LPS to mCD14, (i) when the labeled LPS is contacted with cells in which mCD14 is expressed, and (ii) when the labeled LPS and a test compound are contacted with cells in which mCD14 is expressed; or

(B) using flow cytometry, measuring (i) MFI value when a labeled LPS is contacted with cells in which mCD14 is expressed; (ii) MFI value when the labeled LPS and a test compound are contacted with cells in which mCD14 is expressed; and (iii) MFI value when the labeled LPS and an anti-CD14 antibody are contacted with cells in which mCD14 is expressed, and calculating the inhibition rate from the following equation (I):

$$\text{Inhibition rate (\%)} = (1 - MFI^1/MFI^2) \times 100 \tag{I}$$

(MFI$^1$ = MFI when a test compound is added - MFI when an anti-CD14 antibody is added;

MFI$^2$ = MFI when a test compound is not added - MFI when an anti-CD14 antibody is added), thereby selecting a compound changing the binding property of LPS to CD14, and then measuring an activity inducing production of TNF-α, IL-1, IL-6, IL-8, NO, superoxide, platelet activating factor, or arachidonate cascade product for the selected compound;

　　(a) selecting a compound having said induction activity for the production as (i) CD14 agonist or (ii) CD14 promoter; and
　　(b) selecting a compound-having no induction activity for the production as (i) CD14 antagonist or (ii) CD14 inhibitor.

Particularly, using a compound library prepared by means of a combinatorial chemistry technique as a test compound, (i) CD14 agonists, (ii) CD14 promoters, (iii) CD14 antagonists, or (iv) CD14 inhibitors can be screened efficiently by performing the above-mentioned screening method. The combinatorial chemistry technique includes per se known techniques or those that will be developed in the future.

(4) A screening kit

The screening kit of the invention contains the above-mentioned labeled LPS, cells in which mCD14 is expressed, etc. The screening kit of the invention is exemplified as follows.

[Reagents for screening]

**[0053]**

　　(i) Buffer solution, etc.
　　　PBS, human serum
　　(ii) LPS
　　　FITC-labeled LPS of which the binding ratio (FITC/LPS) is 0.3.
　　(iii) Cells
　　　1,25-Dihydroxyvitamin D$_3$-treated THP-1 which has expressed about 1,000,000 molecules of mCD14 per cell
　　(iv) Others
　　　Anti-human CD14 monoclonal antibody (MEM18)
　　(v) Measurement
　　　The amount of FITC-labeled LPS bound to 1,000 cells is measured by means of a flow cytometer such as CytoACE-300, etc.

(5) Compounds changing the binding property of LPS to CD14

The compounds which are obtainable using the screening method or kit of the invention include those selected from the above-mentioned test compounds, for example, peptides, proteins, non-peptide compounds,. synthetic compounds, fermentation products, cell extracts, plant extracts, animal tissue extracts, plasma, etc., and those which change the binding property of LPS to CD14.

As the derivatives of the compounds which are obtainable using the screening method or kit of the invention, (i) compounds derived from said compounds by deletion of (an) optional substituent(s) in their chemical structure, (ii) compounds derived from said compounds by addition of (an) optional substituent(s) to their chemical structure, (iii) compounds derived from said compounds by conversion of (an) optional substituent(s) in their chemical structure into (an)other substituent(s), (iv) prodrugs of said compounds, etc. are used.

The prodrugs of the compounds (hereinafter, sometimes referred to as the compounds of the invention) which are obtainable using the screening method or kit of the invention, may be compounds which are converted into the compounds of the invention on reaction of an enzyme or gastric acid in a physiological condition in the living body, that is, compounds which are converted into the compounds of the invention by enzymatic oxidation, reduction or hydrolysis or converted into the compounds of the invention by hydrolysis with gastric acid, etc.

As the prodrugs of the compounds of the invention, compounds derived from the compounds of the invention of which the amino group is acylated, alkylated, or phosphorylated (e.g., compounds derived from the compounds of the invention of which the amino group is substituted by eicosanoyl, aranyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl, etc.); compounds derived from the compounds of the invention of which the hydroxy group is acylated, alkylated, phosphorylated, or borylated (e.g., compounds derived from the compounds of the invention of which the hydroxy group is substituted by acetyl, palmitoyl, propanoyl, pivaloyl, succinyl, fumaryl, alanyl, dimethylaminomethyl-carbonyl, etc.); compounds derived from the compounds of the invention of which the carboxyl group is esterified or amidated (e.g., compounds derived from the compounds of the invention of which the carboxyl group is esterified by ethyl, phenyl, carboxymethyl, dimethylaminomethyl, pivaloyloxymethyl, ethoxycarbonyloxyethyl, phthalidyl, (5-methyl-2-oxo-1,3-dioxolen-4-yl)methyl, cyclohexyloxycarbonylethyl, etc., or methylamidated) are exemplified. These compounds may be produced from the compounds of the invention according to a per se known method.

In addition, the prodrugs of the compounds of the invention may be those that can be converted into the compounds of the invention in such a physiological condition as described in "Iyakuhin no Kaihatu" (Development of Drugs), Vol. 7, Molecular Design, pp. 163-198 (1990), Hirokawa Publishing Co.

The compounds or their derivatives which are obtainable using the screening method or kit of the invention, may form salts. As such salts, pharmaceutically and physiologically acceptable salts are used, including, for example, salts with inorganic bases, salts with organic bases, salts with inorganic acids, salts with organic acids, salts with basic or acidic amino acids, etc. As the salts with inorganic bases, for example, alkali metal salts, e.g., sodium salts, potassium salts, etc.; alkaline earth metal salts, e.g., calcium salts, magnesium salts, etc.; as well as aluminum salts, ammonium salts, etc., are preferably used. As the salts with organic bases, for example, salts with trimethylamine, triethylamine, pyridine, picoline, ethanolamine, diethanolamine, triethanolamine, dicyclohexylamine, N,N'-dibenzylethylenediamine, etc. are preferably used. As the salts with inorganic acids, for example, salts with hydrochloric acid, hydrobromic acid, nitric acid, sulfuric acid, phosphoric acid, etc. are preferably used. As the salts with organic acids, for example, salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc. are preferably used. As the salts with basic amino acids, for example, salts with arginine, lysine, ornithine, etc. are preferably used. As the salts with acidic amino acids, for example, salts with aspartic acid, glutamic acid, etc. are preferably used.

Among the compounds or their derivatives which are obtainable using the screening method or kit of the invention, CD14 antagonists or CD14 inhibitors are useful in prevention or treatment of diseases caused by the binding of LPS to mCD14. Such diseases include, for example, heart disease, autoimmune disease, inflammatory disease, disease in central nervous system, infectious disease, sepsis, septic shock, etc. More particularly, such diseases include, for example, sepsis, endotoxin shock, exotoxin shock, heart failure, shock, hypotension, rheumatoid arthritis, arthrosteitis, gastritis, chronic ulcerative colitis, peptic ulcer, stress gastric ulcer, Crohn's disease, autoimmune disease, tissue disturbance and rejection after organ transplantation, ischemic reperfusion injury, acute coronary microvascular embolus, shock vascular embolus (disseminated intravascular coagulation (DIC), etc.), ischemic cerebropathy, arteriosclerosis, pernicious anemia, Fanconi's anemia, sickle cell anemia, pancreatitis, nephrotic syndrome, nephritis, renal failure, insulin-dependent diabetes mellitus, non-insulin-dependent diabetes mellitus, hepatic porphyria, alcoholism, Parkinson's disease, chronic leukemia, acute leukemia, tumor, myeloma, relief of side effect of anti-tumor agents, infant and adult respiratory distress syndrome, pulmonary emphysema, dementia, Alzheimer's disease, multiple sclerosis, vitamin E deficiency, aging, sunburn, muscular dystrophy, myocarditis, cardiomyopathy, myocardial infarction, myocardial infarction sequela, osteoporosis, pneumonia, hepatitis, psoriasis, pain, cataract, influenza infection, malaria, human immunodeficiency virus (HIV) infection, radiation injury, burn, increase of efficiency of in vitro fertilization, hypercalcemia, ankylosing spondylitis, osteopenia, bone Behçet disease, osteomalacia, fracture, acute bacterial meningitis, Helicobacter pylori infection, invasive Staphylococcus infection, tuberculosis, systemic mycosis, Herpes simplex virus infection, herpes zoster varicellosus, human papilloma virus infection, acute viral encephalitis, encephalitis, asthma, atopic dermatitis, allergic rhinitis, reflux esophagitis, fever, hypercholesterolemia, hyperglyceridemia, hyperlipemia, complications of diabetes mellitus, diabetic nephropathy, diabetic neuropathy, diabetic retinopathy, gout, gastric atony, hemorrhoids, systemic lupus erythematosus, spinal cord injury, insomnia, schizophrenia, epilepsy, liver cirrhosis, hepatic insufficiency, unstable angina pectoris, valvular disease of the heart, thrombocytopenia caused by dialysis, acute ischemic cerebral lesion, acute phase cerebral thrombosis, metastatic carcinoma, tumor of the bladder, breast cancer, cancer of the uterine cervix, cancer of the large intestine, gastric cancer, ovarian cancer, prostatic cancer, parvicellular lung cancer, non-parvicellular lung cancer, malignant melanoma, Hodgkin's disease, non-Hodgkin's lymphoma, etc. Particularly, they are useful in prevention or treatment of septic shock.

On the other hand, among the compounds or their derivatives which are obtainable using the screening method or kit of the invention, CD14 promoters or CD14 agonists are useful, for example,-as preventive or therapeutic agents in immunodeficiency or for infections during administration of immunosuppressants or as immune potentiators.

When the compounds or their derivatives or salts which are obtainable using the screening method or kit of the invention are used as therapeutic or preventive agents, it may be performed in a conventional manner. Said compounds or their derivatives or salts may be administered as such or in forms of pharmaceutical compositions. The pharmaceutical compositions used for the administration comprise the above-mentioned compounds or their derivatives and pharmacologically acceptable carriers, diluents or fillers. Such compositions are provided in formulation suitable for oral or parenteral administration.

That is, as the oral compositions, for example, solid or liquid pharmaceutical preparations such as tablets (including sugar-coated tablets, film-coating tablets), pills, granules, powders, capsules (including soft capsules), syrups, emulsions, suspensions, etc. are exemplified. Such compositions may be produced in a per se known method, and contain carriers, diluents or fillers usually employed in the pharmaceutical field. For example, as carriers or fillers for tablets, lactose, starch, sucrose, magnesium stearate, etc. are used.

As the parenteral compositions, for example, injections, suppositoria, etc. are exemplified. The injections include dosage forms such as intravenous injection, subcutaneous injection, intracutaneous injection, intramuscular injection, intravenous drip infusion, etc. Such injections may be prepared in a per se known method, for example, by dissolving, suspending or emulsifying said compound or its derivative or salt in a sterile aqueous or oily solution. As an aqueous solution for injections, for example, physiological saline, isotonic solution containing glucose or other adjuvants, etc. are used. An appropriate solubilizing agent, for example, alcohol (e.g., ethanol), polyalcohol (e.g., propylene glycol, polyethylene glycol), non-ionic surface active agent [e.g., polysorbate 80, HCO-50 (polyoxyethylene (50 mol) adduct of hydrogenated castor oil)], etc. may be used together. As an oily solution, for example, sesame oil, soybean oil, etc. is used. Benzyl benzoate, benzyl alcohol, etc. may be used together as a solubilizing agent. Thus prepared injections are placed in proper ampoules. The suppositoria used in rectal application may be prepared by adding the above-mentioned compound or a derivative or salt thereof to a conventional base for suppositories.

For the above-mentioned oral or parenteral pharmaceutical compositions, it is appropriate to formulate into a unit of dosage form suitable for a dose of the active component. Such a unit of dosage form is exemplified by tablets, pills, capsules, injections (ampoules), suppositories, etc. The unit of dosage form preferably contains usually 5 - 500 mg of the above-mentioned compound or a derivative or salt thereof per dosage form, particularly 5 - 100 mg for injections, and 10 - 250 mg for other dosage forms.

Moreover, the above-mentioned respective compositions may contain (an) additional active component(s) as far as they do not generate adverse effects in combination with the above-mentioned compound or a derivative or salt thereof.

Thus resulting pharmaceutical preparations are safe and less toxic, and may be administered to, for example, mammals (e.g., murine, rat, rabbit, sheep, swine, bovine, bird, feline, canine, monkey, chimpanzee, human, etc.).

The dose of said compounds or derivatives or salts thereof depends on the action, the target disease, the subject to be administered, route of administration. For example, when a CD14 antagonist or CD14 inhibitor is orally administered for treatment of septic shock, they may be given generally in a daily dose of about 0.1 - 100 mg, preferably about 1.0 - 50 mg, more preferably about 1.0 - 20 mg for an adult (as 60 kg body weight).

When said CD14 antagonist or CD14 inhibitor is parenterally administered, á dose varies depending on the subject to be administered, the target disease, etc. For example, when said CD14 antagonist or CD14 inhibitor is usually administered as injection to an adult (as 60 kg body weight) for treatment of septic shock, they may preferably be administered as intravenous injection at a daily dose of about 0.01 - 30 mg, preferably about 0.1-20 mg, more preferably about 0.1 - 10 mg. For other animals, they may be given at a dose calculated from the dose for 60 kg.

On the other hand, when a CD14 agonist or CD14 promoter is orally administered as immunopotentiator, they may be given generally at a daily dose of about 0.1 - 100 mg, preferably about 1.0 - 50 mg, more preferably about 1.0 - 20 mg, for an adult (as 60 kg body weight).

When said CD14 agonist or CD14 promoter is parenterally administered, a dose varies depending on the subject to be administered, the target disease, etc. For example, when said CD14 agonist or CD14 promotor is usually administered as injection to an adult (as 60 kg body weight) as immunopotentiator, they may preferably be administered as intravenous injection at a daily dose of about 0.01-30 mg, preferably about 0.1 - 20 mg, more preferably about 0.1 - 10 mg. For other animals, they may be given at a dose calculated from the dose for 60 kg.

The invention will further be explained more fully by means of the following Reference Examples and Working Examples, but they are not intended to limit the scope of the invention. Moreover, the invention is variable as long as such variation does not deviate from the scope of the invention.

Examples

Reference Example 1

Preparation of FITC-labeled LPS

First, 1 mg of Salmonella minnesota Re595 LPS (Sigma Chemical Co.) was dissoluved in 500 µl of triethylamine (Wako Pure Chemical Ind., Ltd.), and the mixture was sonicated on a multi-sonicating washer VS-III (Iuchi Sei-ei-do Co.) in an ice water bath for 15 minutes. Then, 50µl of 100 mM ethylenediamine tetraacetate (EDTA) was added, and the mixture was further sonicated in an ice water bath for 5 minutes. After sonication, 22.5 µl of 1N hydrochloric acid was added to the reaction mixture, and after confirmation that the pH decreased to 5 or lower, the mixture was allowed to stand at room temperature for 2 minutes and then neutralized with addition of 1N sodium hydroxide. A solution of 5 mg of FITC isomer I (Dojin Iyaku Co., Ltd.) dissolved in 200 µl of 0.5M carbonate/bicarbonate buffer solution (pH 9.5) was added to the reaction mixture, which was again sonicated in an ice water bath for 1 minute. After sonication, 250 µl of 1.6 % sodium deoxycholate (DOC; Wako Pure Chemical Ind., Ltd.) solution was added, the reaction vessel was shaded from light by covering with an aluminum foil, and the mixture was incubated with stirring at 37°C for 18 hours. After completion of the incubation, the mixture volume was adjusted to 2.5 ml with distilled water, and the mixture was applied to gel filtration with a PD-10 column packed with Sephadex G-25M (Pharmacia Biotech Co.) to remove free FITC. Briefly, after the reaction mixture was applied, 3 ml of buffer solution for elution was applied to the column, the fraction eluted immediately after the buffer was applied to the column was collected as a fraction containing FITC-labeled LPS. As a buffer solution for equilibration and elution, Dulbecco's PBS (without $Mg^{2+}$ and $Ca^{2+}$; Dainippon Pharmaceutical Co., Ltd.) - to which DOC was added at a final concentration of 0.16% was used.

The FITC amount in the resulting FITC-labeled LPS was determined at an excitation wavelength of 485 nm and measuring wavelength of 538 nm using a fluorophotometer Fluoroskan II (Labsystems Co.). It was found that 36.18 µg of FITC was bound for 1 mg of LPS. On the basis of the molecular weight, i.e., 389.4 for FITC isomer I and 3,400 for S. minnesota Re595, the binding ratio of FITC to LPS in the FITC-labeled LPS prepared above was about 1 : 3. Reference Example 2

Induction of CD14 expression in THP-1 cells

Human monocytic THP-1 cells (purchased from Dainippon Pharmaceutical Co., Ltd.) were used as cells. As a culture medium, RPMI medium 1640 with L-glutamine (RPMI; GIBCO Co.), to which inactivated fetal calf serum (GIBCO Co.) at a concentration of 10% (v/v) and penicillin-streptomycin involving penicillin G sodium and streptomycin sulfate at a concentration of 100 U/ml and 100 µg/ml respectively, were added. For induction of CD14 expression, the cells were suspended in the medium at a content of about $3 \times 10^5$ cells/ml, to which 1,25-dihydroxyvitamin $D_3$ (VD$_3$; Roussel-UCLAF S.A.) was added at a final concentration of about 100 nM. The cells were incubated in the presence of 5% $CO_2$ in a $CO_2$ incubator MODEL 6200-03 (National Appliance Company) at 37°C for 3 days. The cultured cells were suspended into PBS at $10^6$ cells/ml, to which FITC-labeled anti-human CD14 monoclonal antibody clone. UCHM1 (Ancell Co.) was added at a concentration of 1.6 µg/ml. The mixture was incubated at 4°C for 1 hour. The cells were washed 3 times with PBS by centrifugation (1,000 rpm, 3 minutes, 20°C), suspended into 1,000 µl of PBS, and filtered through a tube with a filter (FALCON2235 (Becton Dickinson Co.)). For the resulting cell suspension, the amount of the FITC-labeled anti-CD14 antibody bound to 10,000 cells was determined using a flow cytometer Cyto ACE-300 (JASCO Coporation). As a buffer solution for elution, FACS Flow (Becton Dickinson Co.) was used.

The obtained value of the mean fluorescence intensity (MFI) was 114.9 for THP-1 cells in which the expression of CD14 was induced with addition of VD$_3$, and 40 for ones in which no VD$_3$ was added. These values appear to indicate the amount of FITC-labeled anti-CD14 antibody bound to 10,000 cells, that is, corresponding to the amount of the expressed CD14. Therefore, the amount of the expressed CD14 markedly increased with addition of VD$_3$.

Example 1

Screening of CD14 antagonist using CD14 expression-inducing THP-1 cells and FITC-labeled LPS

(1) Determination of the activity for inhibiting the binding of FITC-labeled LPS using CD14 expression-inducing THP-1 cells

THP-1 cells in which CD14 expression were induced were washed 3 times with PBS by centrifugation (1,000 rpm, 3 minutes, 20°C), suspended into PBS at a final concentration of $6.67 \times 10^6$ cells/ml, and seeded 150 µl each in a multi-filtration system 96 well plate (Millipore Co.) (final concentration $10^6$ cells/well). Test compound was added 20 µl each to the wells and pre-incubated in the presence of 5% $CO_2$ at 37°C for 15 minutes. To determine the maximum binding, 20 µl each of PBS was added, while an anti-human CD14 monoclonal antibody clone. MEM18 (SANBIO Co.) diluted 10-fold with PBS was added 20 µl each to determine the specific binding to mCD14. After preincubation, 20 µl of FITC-labeled LPS of 0.25 µg/ml concentration (final concentration 5 ng/$10^6$ cells) and 10 µl of human serum (Bio Whittaker Co.) diluted 10-fold with PBS (final concentration 1%) were added to each well, and incubated in the presence of 5% $CO_2$ at 37°C for 1 hour. The cells were washed 3 times with PBS using a Multi-Screen Filtration System Vacuum Manifold (Millipore Co.), then suspended into 300 µl of PBS, and filtered through a tube with a filter. The resulting cell suspension was measured for the amount of FITC-labeled LPS bound to 1,000 cells using a flow cytometer such as CytoACE-300. FACS Flow was used as a buffer solution for elution. The resulting MFI value was regarded as the amount of the FITC-labeled LPS bound to the cells, and the activity of each test compound inhibiting the specific binding property of the FITC-labeled LPS to mCD14 was calculated from the above equation (I).

(2) Determination of the activity for inhibiting the TNF-$\alpha$ production using THP-1 cells

THP-1 cells were washed 3 times with RPMI by centrifugation (1,000 rpm, 3 minutes, 20°C), suspended into PBS at a final concentration of $6.67 \times 10^6$ cells/ml, and seeded 150 µl each in a multi-filtration system 96 well plate (Millipore Co.) (final concentration $10^6$ cells/well). Test compound was added 20 µl each to the wells and pre-incubated in the presence of 5% $CO_2$ at 37°C for 15 minutes. 20 µl each of RPMI was added in reference wells. After pre-incubation, 20 µl of Escherichia coli 0111:B4 LPS (Sigma Chemical Co.) of 0.25 µg/ml concentration (final concentration 5 ng/$10^6$ cells) and 10 µl of human serum (Bio Whittaker Co.) diluted 10-fold with RPMI (final concentration 1%) were added to each well, and incubated in the presence of 5% $CO_2$ at 37°C for 4 hour. After incubation, the supernatant was recovered using a Multi-Screen Filtration System Vacuum Manifold (Millipore Co.). The TNF-$\alpha$ content in the resulting supernatant of the culture medium was determined using an ELISA kit (Genzyme Co.) for determination of human TNF-$\alpha$.

(3) Determination for cytotoxicity using THP-1 cells

THP-1 cells were washed 3 times with 10% inactivated fetal calf serum RPMI by centrifugation (1,000 rpm, 3 minutes, 20°C), suspended into the same culture medium at a final concentration of $10^6$ cells/ml, and seeded 90 µl each in a 96 well microwell plate (ICN Co.) (final concentration $9 \times 10^4$ cells/well). Test compound was added 10 µl each to the wells and incubated in the presence of 5% $CO_2$ at 37°C for 24 hours. 10 µl each of RPMI was added in reference wells. After incubation, the presence of cytotoxicity was examined using cell counting kit (Dojin Iyaku Co., Ltd.). Briefly, 10 µl of the coloring agent provided in the kit was added to each well, and incubated in the presence of 5% $CO_2$ at 37°C for 3 hours. Then, the absorbance was measured at a measuring wavelength of 450 nm and a reference wavelength of 620 nm.

The inhibitory activity of the binding was determined for 10 species of compounds at a concentration of 2 µM using the above-mentioned method (1). The result was as shown in Table 1.

Table 1

| Compound No. | Binding Inhibition Rate (%) |
|---|---|
| 1 | 61.9 |
| 2 | 56.4 |
| 3 | 51.3 |
| 4 | 51.9 |
| 5 | 60.4 |
| 6 | 56.9 |

Table 1 (continued)

| Compound No. | Binding Inhibition Rate (%) |
|---|---|
| 7 | 66.1 |
| 8 | 55.4 |
| 9 | 50.8 |
| 10 | 50.3 |

From Table 1, it was confirmed that Compounds 1 to 10 exhibit a potent inhibitory activity against the binding of LPS to CD14.

Moreover, a variety of in vitro activities were determined on 3 species of compounds using the above-mentioned methods (1) - (3). Table 2 shows the result.

Table 2

| Compound No. | Inhibition of binding of FITC-labeled LPS | Inhibition of TNF-$\alpha$ Production | Cytotoxicity |
|---|---|---|---|
| | $IC_{50}$ | $IC_{50}$ | $IC_{50}$ |
| A | 4.39 $\mu$M | 4.79 $\mu$M | >10 $\mu$M |
| B | 4.39 $\mu$M | 10.39 $\mu$M | >10 $\mu$M |
| C | 4.26 $\mu$M | 9.21 $\mu$M | >10 $\mu$M |

From Table 2, it was confirmed that the compounds A - C strongly inhibit the binding of LPS to CD14 and have an inhibitory activity of TNF-$\alpha$ production, i.e., activity as the so-called CD14 antagonist.

Industrial Applicability

The screening method and kit of the invention are useful in screening compounds which change the binding property of LPS to CD14. The compounds or their derivatives which can be identified by said screening to change the binding property of LPS to CD14 are useful as agents for prevention or treatment of septic shock, etc., or as immunopotentiators.

**Claims**

1. A method for screening compounds changing the binding property of lipopolysaccharide to CD14 which comprises using a labeled lipopolysaccharide and cells in which a membrane-bound CD14 is expressed.

2. A method for screening according to Claim 1, wherein lipopolysaccharide is one labeled with fluorescein isothiocyanate, biotin, enzyme or radioactive isotope.

3. A method for screening according to Claim 1, wherein lipopolysaccharide is a rough form of lipopolysaccharide.

4. A method for screening according to Claim 1, wherein lipopolysaccharide is one labeled with fluorescein isothiocyanate.

5. A method for screening according to Claim 1, wherein lipopolysaccharide labeled with fluorescein isothiocyanate in which the binding ratio (fluorescein isothiocyanate/ lipopolysaccharide) is about 0.03 to about 1.0, and cells in which membrane-bound CD14 is highly expressed are used.

6. A method for screening according to Claim 1 or 5, wherein cells in which about 100,000 or more molecules of membrane-bound CD14 per cell is expressed are used.

7. A method for screening according to Claim 1, wherein the cells are those in which the expression of membrane-bound CD14 is induced by 1,25-dihydroxyvitamin $D_3$.

8. A method for screening according to Claim 1, wherein the cells are mononuclear leukocytes or polymorphonuclear leukocytes.

9. A method for screening according to Claim 1, wherein the cells are monocytes, macrophages or neutrophils.

10. A method for screening according to Claim 1, wherein the cells are human cells.

11. A method for screening according to Claim 1, wherein the cells are THP-1.

12. A method for screening according to Claim 1, wherein about 1 ng to about 30 ng of labeled lipopolysaccharide for about $10^6$ cells are used;

13. A method for screening according to Claim 1, which comprises measuring the binding property of a labeled lipopolysaccharide to membrane-bound CD14, (i) when the labeled lipopolysaccharide is contacted with cells in which membrane-bound CD14 is expressed, and (ii) when the labeled lipopolysaccharide and a test compound are contacted with cells in which membrane-bound CD14 is expressed.

14. A method for screening compounds changing the binding property of lipopolysaccharide to CD14 which comprises using flow cytometry, measuring (i) values of mean fluorescence intensity (MFI) when a labeled lipopolysaccharide is contacted with cells in which membrane-bound CD14 is expressed; (ii) MFI value when the labeled lipopolysaccharide and a test compound are contacted with cells in which membrane-bound CD14 is expressed; and (iii) MFI value when the labeled lipopolysaccharide and an anti-CD14 antibody are contacted with cells in which membrane-binding CD14 is expressed, and calculating the inhibition rate from the following equation (I):

$$\text{Inhibition rate (\%)} = (1 - \text{MFI}^1/\text{MFI}^2) \times 100 \qquad (I)$$

($\text{MFI}^1$ = MFI when a test compound is added - MFI when an anti-CD14 antibody is added;
$\text{MFI}^2$ = MFI when a test compound is not added - MFI when an anti-CD14 antibody is added).

15. A method for screening according to Claim 1, wherein the compound changing the binding property of lipopolysaccharide to CD14 is a CD14 antagonist.

16. A method for screening according to Claim 1, wherein the compound changing the binding property of lipopolysaccharide to CD14 is a CD14 agonist.

17. A method for screening (i) CD14 agonists, (ii) CD14 antagonists, (iii) compounds which are not bound to membrane-bound CD14 but act on lipopolysaccharide, CD14 or lipopolysaccharide-binding protein to inhibit the binding of lipopolysaccharide to CD14, or (iv) compounds which promote the binding of lipopolysaccharide to CD14, which comprises measuring the presence of the lipopolysaccharide-like action of compounds obtained by the method for screening according to Claim 13 or 14.

18. A method for screening according to Claim 17, wherein the lipopolysaccharide-like action is production of TNF-$\alpha$, IL-1, IL-6, IL-8, NO, superoxide, platelet activating factor, or arachidonate cascade product.

19. A kit for screening compounds changing the binding property of lipopolysaccharide to CD14 which comprises a labeled lipopolysaccharide and cells in which membrane-bound CD14 is expressed.

20. A compound or derivative thereof changing the binding property of lipopolysaccharide to CD14, which is obtainable according to the method for screening according to Claim 1.

21. (i) a CD14 agonist, (ii) a CD14-antagonist, (iii) a compound which is not bound to membrane-bound CD14 but acts on lipopolysaccharide, CD14 or lipopolysaccharide-binding protein to inhibit the binding of lipopolysaccharide to CD14, or (iv) a compound which promotes the binding of lipopolysaccharide to CD14, or a derivative thereof, which is obtainable according to the method for screening according to Claim 17.

22. An agent for preventing or treating septic shock which comprises a compound or derivative thereof changing the binding property of lipopolysaccharide to CD14, which is obtainable according to the method for screening according to Claim 1.

**23.** An agent for preventing or treating septic shock according to Claim 22, wherein the compound is a CD14 antagonist.

**24.** An agent for preventing or treating septic shock according to Claim 22, wherein the compound is one which is not bound to membrane-bound CD14 but acts on lipopolysaccharide, CD14 or lipopolysaccharide-binding protein to inhibit the binding of lipopolysaccharide to CD14.

**25.** An immunopotentiator which comprises a compound or derivative thereof changing the binding property of lipopolysaccharide to CD14, which is obtainable according to the method for screening according to Claim 1.

**26.** An immunopotentiator according to Claim 25, wherein the compound is a CD14 agonist.

**27.** An immunopotentiator according to Claim 25, wherein the compound is one which promotes the binding of lipopolysaccharide to CD14.

**28.** A method for preventing or treating septic shock which comprises administering an effective amount of a compound or derivative thereof changing the binding property of lipopolysaccharide to CD14 to mammals, which is obtainable according to the method for screening according to Claim 1.

**29.** A method for immunopotentiation which comprises administering an effective amount of a compound or derivative thereof changing the binding property of lipopolysaccharide to CD14 to mammals, which is obtainable according to the method for screening according to Claim 1.

**30.** Use of a compound or derivative thereof changing the binding property of lipopolysaccharide to CD14 for preparing an agent for preventing or treating septic shock, which is obtainable according to the method for screening according to Claim 1.

**31.** Use of a compound or derivative thereof changing the binding property of lipopolysaccharide to CD14 for preparing an agent for immunopotentiation, which is obtainable according to the method for screening according to Claim 1.

**32.** A method for screening (i) CD14 agonist, (ii) a compound promoting the binding of lipopolysaccharide to CD14, (iii) CD14 antagonist, or (iv) a compound which is not bound to membrane-bound CD14 but acts on lipopolysaccharide, CD14 or lipopolysaccharide-binding protein to inhibit the binding of lipopolysaccharide to CD14, which comprises:

(A) measuring the binding property of the labeled lipopolysaccharide to membrane-bound CD14, (i) when the labeled lipopolysaccharide is contacted with cells in which membrane-bound CD14 is expressed, and (ii) when the labeled lipopolysaccharide and a test compound are contacted with cells in which membrane-bound CD14 is expressed; or
(B) using flow cytometry, measuring (i) values of mean fluorescence intensity (MFI) when a labeled lipopolysaccharide is contacted with cells in which membrane-bound CD14 is expressed; (ii) MFI value when the labeled lipopolysaccharide and a test compound are contacted with cells in which membrane-bound CD14 is expressed; and (iii) MFI value when the labeled lipopolysaccharide and an anti-CD14 antibody are contacted with cells in which membrane-bound CD14 is expressed, and calculating the inhibition rate from the following equation (I):

$$\text{Inhibition rate (\%)} = (1 - MFI^{1}/MFI^{2}) \times 100 \qquad (I)$$

($MFI^{1}$ = MFI when a test compound is added - MFI when an anti-CD14 antibody is added;
$MFI^{2}$ = MFI when a test compound is not added - MFI when an anti-CD14 antibody is added), thereby selecting a compound changing the binding property of lipopolysaccharide to CD14, and then measuring an activity inducing production of TNF-$\alpha$, IL-1, IL-6, IL-8, NO, superoxide, platelet activating factor, or arachidonate cascade product for the selected compound;

(a) selecting a compound having said induction activity for the production as (i) CD14 agonist or (ii) a compound promoting the binding of lipopolysaccharide to CD14; and
(b) selecting a compound having no said induction activity for the production as (i) CD14 antagonist or (ii) a compound which is not bound to membrane-bound CD14 but acts on lipopolysaccharide, CD14 or lipopolysaccharide-binding protein to inhibit the binding of lipopolysaccharide to CD14.

**33.** A compound or derivative thereof selected from a compound library prepared by means of a combinatorial chemistry technique according to the method for screening according to Claim 32, that is, (i) CD14 agonist, (ii) a compound promoting the binding of lipopolysaccharide to CD14, (iii) CD14 antagonist, or (iv) a compound which is not bound to membrane-bound CD14 but acts on lipopolysaccharide, CD14 or lipopolysaccharide-binding protein to inhibit the binding of lipopolysaccharide to CD14.

## INTERNATIONAL SEARCH REPORT

| International application No. |
|---|
| PCT/JP99/06537 |

| A. CLASSIFICATION OF SUBJECT MATTER |
|---|
| Int.Cl⁷   C12Q1/02, A61K45/00, A61P31/00, 37/04 |

According to International Patent Classification (IPC) or to both national classification and IPC

| B. FIELDS SEARCHED |
|---|
| Minimum documentation searched (classification system followed by classification symbols)<br>    Int.Cl⁷   C12Q1/00-3/00 |
| Documentation searched other than minimum documentation to the extent that such documents are included in the fields searched |
| Electronic data base consulted during the international search (name of data base and, where practicable, search terms used)<br>    WPI (DIALOG), BIOSIS (DIALOG), MEDLINE (STN) |

### C. DOCUMENTS CONSIDERED TO BE RELEVANT

| Category* | Citation of document, with indication, where appropriate, of the relevant passages | Relevant to claim No. |
|---|---|---|
| X | The Journal of Biological Chemistry, Volume 273, Number 15, issued April 10, 1998, Roman Dziarski et al., "Binding of  Bacterial Peptidoglycan to CD14", pages 8680-8690 | 20,21,33 |
| X | Journal of Applied Physiology, Volume 76, Number 4, issued April 1994, H. Hank Simms and R. D'Amico, "Hypoxemia regulates effect of lipopolysaccharide on polymorphonuclear leukocyte CD11b/CD18 expression", pages 1657-1663 | 20,21,33 |
| X | Annual Review of Immunology, Volume 13, issued 1995, R.J. Ulevitch and P.S. Tobias, "Receptor-dependent mechanisms of cell stimulation by bacterial endotoxin", pages 437-457 | 20,21,33 |
| X | The Journal of Biological Chemistry, Volume 273, Number 38, issued September 18, 1998, Ping-yuan Wang et al., "Phospha- tidylinositides Bind to Plasma Membrane CD14 and Can      Prevent Monocyte Activation by Bacterial Lipopolysaccha-   ride", pages 24309-24313 | 20,21,33 |
| A | Database MEDLINE on STN, Accession No.95202576, Pugin,J. | 1-27,30-33 |

☒ Further documents are listed in the continuation of Box C.　　☐ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 March, 2000 (08.03.00) | 21 March, 2000 (21.03.00) |

| Name and mailing address of the ISA/ | Authorized officer |
|---|---|
|     Japanese Patent Office | |
| Facsimile No. | Telephone No. |

Form PCT/ISA/210 (second sheet) (July 1992)

EP 1 134 289 A1

**INTERNATIONAL SEARCH REPORT**

International application No.

PCT/JP99/06537

C (Continuation). DOCUMENTS CONSIDERED TO BE RELEVANT

| Category* | Citation of document, with indication, where appropriate, of the relevant passages | Relevant to claim No. |
|---|---|---|
| | et al., "CD14 is a pattern recognition receptor.", Immunity, Volume 1, Number 6, issued September 1994, pages 509-516 | |
| A | Clinical Infectious Diseases, Volume 18, Supplement 2, issued February 1994, M.P. Glauser et al., "Pathogenesis and Potential Strategies for Prevention and Treatment of Septic Shock: An Update", pages S205-S216 | 1-27,30-33 |
| A | Infection and Immunity, Volume 65, Number 6, issued June 1997, Annet Troelstra et al., "Saturable CD14-Dependent Binding of Fluorescein-Labeled Lipopolysaccharide to Human Monocytes", pages 2272-2277 | 1-27,30-33 |
| A | Infection and Immunity, Volume 66, Number 2, issued February 1998, Elisabeth Elass-Rochard et al., "Lactoferrin Inhibits the Endotoxin Interaction with CD14 by Competition with the Lipopolysaccharide-Binding Protein", pages 486-491 | 1-27,30-33 |
| A | Infection and Immunity, Volume 65, Number 3, issued March 1997, B. Weidemann et al., "Specific Binding of Soluble Peptidoglycan and Muramyldipeptide to CD14 on Human Monocytes", pages 858-864 | 1-27,30-33 |
| A | Clinical Infectious Diseases, Volume 21, Supplement 2, issued October 1995, Suganya Viriyakosol and Theo Kirkland, "Knowledge of Cellular Receptors for Bacterial Endotoxin--- 1995", pages S190-S195 | 1-27,30-33 |

Form PCT/ISA/210 (continuation of second sheet) (July 1992)

19

**EP 1 134 289 A1**

## INTERNATIONAL SEARCH REPORT

| International application No. |
|---|
| PCT/JP99/06537 |

**Box I    Observations where certain claims were found unsearchable (Continuation of item 1 of first sheet)**

This international search report has not been established in respect of certain claims under Article 17(2)(a) for the following reasons:

1. ☒ Claims Nos.: 28,29
   because they relate to subject matter not required to be searched by this Authority, namely:

   Claim 28 pertains to preventive/therapeutic method for septic shock in mammals, while claim 29 pertains to method of immunopotentiation of mammals. These claims each pertains to methods for treatment of the human or animal body by therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II    Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.
                         ☐    No protest accompanied the payment of additional search fees.

Form PCT/ISA/210 (continuation of first sheet (1)) (July 1992)

20